Europäisches Patentamt

European Patent Office

Office européen des brevets

(19)

(11) Numéro de publication : **0 135 442**
B1

# (12) FASCICULE DE BREVET EUROPÉEN

(45) Date de publication du fascicule du brevet :
30.12.86

(21) Numéro de dépôt : **84401785.5**

(22) Date de dépôt : **11.09.84**

(51) Int. Cl.⁴ : **C 07 C   5/08**, C 07 C   7/167,
C 07 C 11/04

(54) **Procédé d'hydrogénation sélective de l'acétylène contenu dansun mélange d'acétylène et d'étylène.**

(30) Priorité : **19.09.83 FR 8314969**

(43) Date de publication de la demande :
**27.03.85 Bulletin 85/13**

(45) Mention de la délivrance du brevet :
**30.12.86 Bulletin 86/52**

(84) Etats contractants désignés :
**BE DE GB IT NL**

(56) Documents cités :
**EP-A- 0 064 301**
**BE-A-   553 873**
**BE-A-   564 339**
**FR-A-   651 037**
**CHEMICAL ABSTRACTS, vol. 95, no. 10, novembre 1981, page 677, no. 168513e, Columbus, Ohio, US.**

(73) Titulaire : **INSTITUT FRANCAIS DU PETROLE**
**4, Avenue de Bois-Préau**
**F-92502 Rueil-Malmaison (FR)**

(72) Inventeur : **Cosyns, Jean**
**50, Route d'Herbeville**
**F-78580 Maule (FR)**
Inventeur : **Boitiaux, Jean-Paul**
**4, Avenue des Ursulines**
**F-78300 Poissy (FR)**

EP 0 135 442 B1

Jouve, 18, rue St-Denis, 75001 Paris, France

## Description

Les hydrocarbures oléfiniques et dioléfiniques produits par les procédés thermiques de conversion tels que le vapocraquage, par exemple, sont toujours associés à des hydrocarbures plus fortement insaturés, en particulier à des hydrocarbures acétyléniques.

Une hydrogénation sélective de ces derniers hydrocarbures permet d'obtenir les spécifications voulues pour les hydrocarbures monooléfiniques et dioléfiniques.

Ces hydrogénations sont faites avec des catalyseurs sélectifs, par exemple du palladium sur alumine.

Néanmoins les procédés catalytiques actuels ne donnent pas entière satisfaction ; les rendements obtenus sont en effet le plus souvent inférieurs à 100 % et la stabilité du catalyseur est généralement insuffisante.

Ces difficultés sont illustrées ci-après en relation avec l'hydrogénation de la coupe $C_2$.

La coupe éthylénique $C_2$ sortant du vapocraqueur a généralement la composition type suivante :

| Acétylène | 1 à 2 % en volume |
|---|---|
| Ethylène | 70 à 90 % en volume |
| Ethane | 10 à 30 % en volume |

La teneur en acétylène de la coupe éthylénique doit être amenée à 2 ppm en volume, ou au-dessous, par hydrogénation sélective de l'acétylène sur catalyseur au palladium. Ce résultat a été obtenu jusqu'à présent par emploi de procédés isothermes ou adiabatiques fonctionnant le plus souvent en phase gazeuse sous environ 20 à 30 bar à une température comprise habituellement entre 60 et 150 °C.

Les inconvénients de ces procédés sont nombreux :

L'exothermicité de la réaction oblige à l'installation de plusieurs réacteurs en série car la phase gazeuse n'est pas favorable à l'élimination des calories. De plus la quantité de catalyseur utilisée est importante ; une vitesse spatiale gazeuse de 2 000 vol/vol/heure est courante (soit 3 tonnes de catalyseur environ pour traiter 10 t/h de coupe $C_2$).

Le catalyseur n'est pas parfaitement sélectif. Le rendement potentiel en éthylène étant de 101 à 102 %, les rendements constatés dépassent difficilement 99,5 %. Ces bas rendements sont dus à deux causes : une sélectivité insuffisante qui conduit à une production excessive d'éthane et une polymérisation parasite de l'acétylène en produits plus ou moins lourds appelés souvent « green oils ».

Ces polymères se déposent sur le catalyseur et réduisent fortement la durée des cycles.

On a déjà décrit pour l'hydrogénation de l'acétylène des procédés utilisant un solvant. C'est le cas du procédé du brevet U.S. 4.128.595 dans lequel il est recommandé d'utiliser un solvant hydrocarboné inerte. L'emploi d'un solvant présente plusieurs avantages par rapport au procédé d'hydrogénation en phase gazeuse et notamment les suivants :

— meilleur contrôle de l'exothermicité de la réaction.

— amélioration de la sélectivité de l'hydrogénation et donc du rendement en éthylène produit.

— amélioration de l'activité et de la stabilité du catalyseur.

L'objet de la présente invention est la mise au point d'un nouveau procédé d'hydrogénation catalytique sélective d'un mélange d'acétylène et d'éthylène qui permet non seulement de travailler dans des conditions douces mais surtout, et de façon surprenante, d'améliorer l'activité, la sélectivité et la durée de vie des catalyseurs.

L'invention est applicable, en particulier, aux coupes éthylène provenant du vapocraquage.

Le procédé de l'invention consiste à effectuer l'hydrogénation sélective en présence d'un catalyseur de palladium sur alumine dans une phase liquide organique (généralement à base d'un ou plusieurs hydrocarbures) ladite phase liquide renfermant 15 à 100 % en poids d'au moins un hydrocarbure aromatique, ladite phase renfermant en outre un composé aminé en solution. On améliore ainsi sensiblement le rendement en éthylène et la stabilité du catalyseur.

Un procédé voisin a été proposé autrefois dans le brevet belge N° 564 339 à l'aide d'un catalyseur au palladium précipité sur un support tel que les carbonates de calcium ou de baryum, le sulfate de baryum, le charbon actif ou le gel de silice, en présence d'une amine de type hétérocyclique qui était la quinoléine ou la pyridine et en présence d'un solvant choisi parmi l'eau, les alcools ou des hydrocarbures non aromatiques.

Or, selon la présente invention, il importe que la phase liquide renferme au moins un hydrocarbure aromatique et par exemple le benzène, le toluène, l'éthylbenzène ou une coupe naphta ou kérosène contenant au moins un hydrocarbure aromatique de façon à ce que dans la phase liquide, utilisée à titre de solvant, la teneur pondérale en hydrocarbure(s) aromatique(s) soit d'au moins 15 %.

On choisit de préférence le (ou les) hydrocarbure(s) de la phase liquide tel qu'il(s) puisse(nt) être facilement séparé(s) des produits de l'hydrogénation ; de préférence, cet hydrocarbure aura un point d'ébullition supérieur à celui de la coupe traitée.

En outre, il importe que l'on utilise une amine ou polyamine en proportion de 0,01 à 10 %, de préférence de 0,1 à 1 % du poids du ou des hydrocarbures que contient la phase liquide. L'amine (ou chaque fonction amine de la polyamine) est primaire ou secondaire, par exemple à chaîne aliphatique ou cyclique : on peut citer par exemple la butylamine, l'éthylamine, la diéthylamine, la triméthylamine, la pipéridine, la morpholine, la pipérazine, l'éthylènediamine et la diéthylènetriamine. L'amine peut renfermer des substituants ou fonctions autres qu'amine, par exemple des groupes alcool ou éther (ainsi la morpholine, l'éthanolamine ou la diéthanolamine). Les amines à structure hétérocyclique (du type quinoléine ou pyridine) ne font pas partie de l'invention.

Le catalyseur d'hydrogénation est constitué par du palladium supporté. Le palladium est en général déposé à raison de 0,01 à 1 % en poids sur un support d'alumine. Au palladium peut être associé un deuxième métal tel que l'argent ou l'or, à des teneurs qui pourront être comprises par exemple entre 0,01 et 1 % du poids du catalyseur. De préférence le rapport pondéral Au/Pd ou Ag/Pd est inférieur à 1. L'or donne des résultats particulièrement intéressants, le rapport pondéral Au/Pd étant compris de préférence entre 0,05 et 0,5.

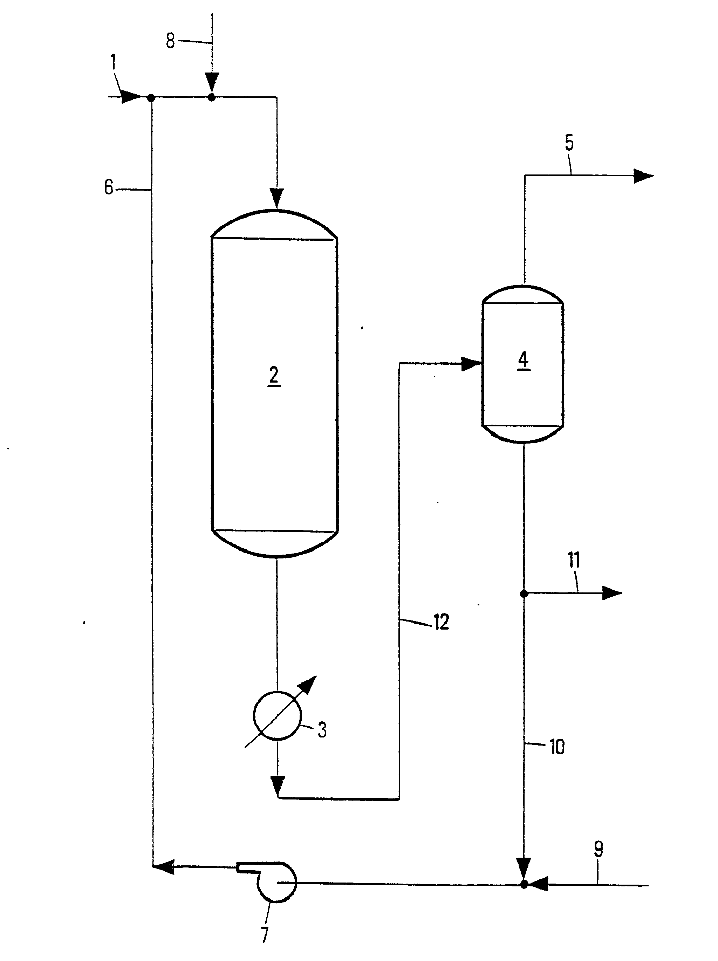

L'hydrogénation peut être réalisée dans un réacteur dans lequel le catalyseur est disposé en lit fixe. La figure jointe présente un exemple d'application de l'invention au cas de l'hydrogénation d'une coupe $C_2$. La coupe à hydrogéner (1), de l'hydrogène (8) et le diluant liquide (6) sont introduits dans le réacteur (2). Après refroidissement dans l'échangeur (3) le mélange liquide-gaz est introduit par la ligne (12) dans le ballon de désengagement (4) qui sépare la coupe purifiée gazeuse (5) du diluant liquide (10) que l'on recycle à travers la pompe (7). La canalisation (11) permet de purger une partie du solvant et la canalisation (9) d'en faire l'appoint.

Les conditions opératoires de l'hydrogénation de la coupe $C_2$ sont les suivantes :

— vitesse spatiale exprimée en débit volumique de coupe $C_2$ gazeuse à température et pression normales par volume de catalyseur et par heure (VVH gaz) : 500 à 20 000, de préférence 1 000 à 10 000.
— pression totale : 10 à 50 bars
— température : 20 à 150 °C.

Le débit d'hydrogène est utilement ajusté en fonction de la teneur en hydrocarbures acétyléniques de la charge. On l'exprime en moles d'hydrogène par mole d'hydrocarbures acétyléniques introduite dans le réacteur. Ce rapport sera généralement compris entre 1 et 10 et de préférence entre 1 et 2.

Le débit de solvant (hydrocarbure + amine) est utilement réglé par rapport au volume du catalyseur. On l'exprime en débit volumique de liquide par volume de catalyseur et par heure (VVH liq). Cette valeur sera généralement comprise entre 1 et 10.

La teneur en amine est réglée entre 0,01 et 10 % du poids d'hydrocarbure de la phase liquide et de préférence entre 0,1 et 2 % de ce poids.

Les exemples suivants, donnés à titre non limitatif, illustrent l'invention.

Exemple 1 (comparatif)

Dans cet exemple qui illustre une technique de l'art antérieur, on traite une coupe gazeuse contenant 99 % en poids d'éthylène et 1 % en poids d'acétylène. On n'utilise pas de diluant liquide. Le catalyseur contient 0,2 % en poids de palladium déposé sur un support d'alumine de surface spécifique égale à 70 m²/g et d'un volume poreux égal à 0,6 cm³/g. Le catalyseur est disposé en lit fixe dans un réacteur tubulaire.

On fait passer la coupe $C_2$ dans ce réacteur dans les conditions suivantes :

VVH gaz = 2 500
Pression = 25 bars
Température = 25 °C
$H_2$/acétylène = 2 moles/mole

La composition de la coupe $C_2$ à la sortie du réacteur est la suivante après 5 heures d'essai.

| | |
|---|---|
| Acétylène | 100 ppm |
| Ethylène | 98,15 % poids |
| Ethane | 1,84 % poids |

Le rendement en éthylène, exprimé en pourcentage d'éthylène sorti par rapport à l'éthylène entré, n'est que de 99,15 %.

On poursuit l'essai dans les mêmes conditions pendant 50 heures. On obtient alors la composition suivante de l'effluent $C_2$ :

| | |
|---|---|
| Acétylène | 0,20 % poids |
| Ethylène | 99,50 % poids |
| Ethane | 0,30 % poids |

On voit que l'activité du catalyseur ne se maintient pas puisque la conversion de l'acétylène n'est plus que de 80 % au bout du temps considéré (teneur en acétylène : 1 % en poids à l'entrée, 0,2 % en poids à la sortie).

## Exemple 2

Dans cet exemple, on traite la même coupe que celle de l'exemple 1. Les conditions opératoires sont les mêmes que dans l'exemple 1 sauf pour le rapport $H_2$/acétylène qui est ajusté pour obtenir la conversion désirée de l'acétylène. Cependant la valeur de ce rapport est restée toujours comprise entre 1,5 et 2 moles/mole.

On ajoute à la coupe $C_2$ un solvant liquide qui passe également sur le lit catalytique à un débit correspondant à VVH liq = 5.

Le solvant est recueilli dans un ballon séparateur et recyclé comme indiqué dans le schéma de l'invention.

Divers solvants ont été utilisés. Le tableau 1 résume les résultats obtenus. On y a rassemblé les rendements en éthylène que l'on obtient en utilisant les différents solvants. La valeur de ce rendement est donnée pour 2 ppm et 100 ppm d'acétylène résiduel.

### Tableau 1

Solvants                                                               Rendement en éthylène %

|  | 100 ppm d'acétylène résiduel | 2 ppm d'acétylène résiduel |
|---|---|---|
| Heptane | 100,38 | 100,22 |
| Diméthylformamide | 100,48 | - (*) |
| Benzène | 100,61 | 100,47 |
| Benzène+0,2% en poids de pipéridine | 100,69 | 100,65 |

* dans ce cas, on n'a pas pu obtenir une teneur en acétylène de 2 ppm même avec un rapport $H_2$/acétylène = 2 moles/mole.

On voit que le solvant benzénique dans lequel on a ajouté 0,2 % poids de pipéridine permet l'obtention du meilleur rendement en éthylène.

Avec la charge utilisée, le rendement théorique est de 101 % en considérant que tout l'acétylène doit se transformer sélectivement en éthylène. Le procédé de l'invention permet donc de convertir en éthylène 65 % de l'acétylène, même à conversion quasi complète de celui-ci.

## Exemple 3

Dans cet exemple, on effectue l'hydrogénation de la même coupe $C_2$ que dans l'exemple 1 avec les divers solvants utilisés dans l'exemple 2. La charge et les conditions opératoires sont les mêmes que dans l'exemple 2. Les essais sont poursuivis pendant 50 heures. On a également opéré en utilisant, à titre d'amine, 0,2 % de quinoléine et 0,2 % de pyridine (amines non conformes à l'invention).

Dans le tableau 2, on a indiqué la teneur résiduelle en acétylène obtenue au bout de la 5e et de la 50e heure.

### Tableau 2

Solvants                                                               Acétylène résiduel (ppm)

|  | 5 heures | 50 heures |
|---|---|---|
| Diméthylformamide | 122 | 4.950 |
| Heptane | 1,5 | 50 |

4

**0 135 442**

(Suite)

|  | 5 heures | 50 heures |
|---|---|---|
| Benzène | 1 | 10 |
| Benzène +0,2% en poids de pipéridine | 1 | 2 |
| Benzène+0,2% en poids de quinoléine | 1,5 | 4 |
| Benzène+0,2% en poids de pyridine | 1,5 | 5 |

On constate que le procédé de l'invention utilisant un hydrocarbure aromatique contenant une amine, permet d'obtenir à la fois la meilleure activité et la meilleure stabilité du catalyseur.

Exemple 4

Dans cet exemple, on effectue l'hydrogénation de la même coupe $C_2$ que dans l'exemple 1.

On ajoute à la coupe $C_2$ divers solvants liquides qui sont respectivement le n-heptane, le toluène et un naphta dont l'intervalle de distillation est 35-170 °C et qui renferme 20 % en poids d'hydrocarbures aromatiques, à un débit correspondant à VVH liq = 5. A chaque solvant on ajoute 0,2 % en poids de pipéridine. Les conditions opératoires sont les mêmes que dans l'exemple 2.

Le tableau 3 résume les résultats obtenus. On y a rassemblé les rendements en éthylène obtenus avec ces différents solvants pour des teneurs en acétylène résiduel de 2 à 100 ppm. On note l'intérêt d'opérer en présence d'un solvant aromatique.

Tableau 3

Solvants                                                                    Rendements en éthylène

|  | 100 ppm d'acétylène | 2 ppm d'acétylène |
|---|---|---|
| Heptane + 0,2 % pipéridine | 100,62 | 100,53 |
| Toluène + 0,2 % pipéridine | 100,72 | 100,69 |
| Naphta (avec 20% d'aromatiques) + 0,2 % pipéridine | 100,68 | 100,63 |

Exemple 5

Dans cet exemple, on effectue l'hydrogénation de la coupe $C_2$ avec les divers solvants utilisés dans l'exemple 4. La charge et les conditons opératoires sont les mêmes que dans l'exemple 2. Les essais sont poursuivis pendant 50 heures. Dans le tableau 4 on a indiqué la teneur résiduelle en acétylène obtenue au bout de la 5e et de la 50e heure.

Tableau 4

Solvants                                                                    Acétylène résiduel (ppm)

|  | 5 heures | 50 heures |
|---|---|---|
| Heptane+0,2% pipéridine | 1 | 4 |
| Toluène+0,2% pipéridine | 1 | 1,5 |
| Naphta +0,2% pipéridine | 1 | 2 |

5

**0 135 442**

### Exemple 6

Dans cet exemple, on effectue l'hydrogénation de la même coupe $C_2$ que dans l'exemple 1, dans les conditions opératoires de l'exemple 2. On ajoute à la coupe $C_2$ du toluène qui passe également sur le lit catalytique à un débit correspondant à VVH liq = 5. A ce toluène on ajoute 0,2 % en poids de divers composés aminés qui sont respectivement la butylamine, la diéthylamine, l'éthanolamine et l'éthylènediamine.

Le tableau 5 résume les résultats obtenus pour des teneurs résiduelles en acétylène de 100 et 2 ppm.

#### Tableau 5

| Solvants | Rendement en éthylène % | |
|---|---|---|
| | 100 ppm d'acétylène résiduel | 2 ppm d'acétylène résiduel |
| Toluène+0,2% butylamine | 100,68 | 100,65 |
| Toluène+0,2% diéthylamine | 100,64 | 100,57 |
| Toluène+0,2% éthanolamine | 100,67 | 100,65 |
| Toluène+0,2% éthylènediamine | 100,70 | 100,67 |

### Exemple 7

Dans cet exemple, on effectue l'hydrogénation de la coupe $C_2$ avec les divers solvants utilisés dans l'exemple 6. La charge et les conditions opératoires sont les mêmes que dans l'exemple 2. Les essais sont poursuivis pendant 50 heures. Dans le tableau 6 on a indiqué la teneur résiduelle en acétylène obtenue au bout de la 5e et de la 50e heure.

#### Tableau 6

| Solvants | Acétylène résiduel (ppm) | |
|---|---|---|
| | 5 heures | 50 heures |
| Toluène + 0,2 % butylamine | 1 | 2 |
| Toluène + 0,2 % diéthylamine | 1 | 3 |
| Toluène + 0,2 % éthanolamine | 1 | 2,5 |
| Toluène + 0,2 % éthylènediamine | 1 | 2 |

### Exemple 8

Dans cet exemple, on effectue l'hydrogénation d'une coupe $C_2$ contenant 98 % en poids d'éthylène et 2 % en poids d'acétylène dans les mêmes conditions que dans les exemples ci-dessus. On utilise le toluène comme solvant auquel on ajoute 0,1 % d'éthylènediamine. L'hydrogénation est effectuée comparativement sur deux catalyseurs conformes à l'invention contenant respectivement 0,2 % de palladium en poids déposé sur alumine et 0,2 % de palladium en poids plus 0,05 % en poids d'or, ces métaux étant déposés sur alumine. Le tableau 7 résume les résultats obtenus lorsque les teneurs résiduelles en acétylène sont respectivement 100 et 2 ppm. On note ainsi que la présence d'or améliore les résultats.

6

# 0 135 442

Tableau 7

Catalyseur                                                                    Rendement en éthylène

| CATALYSEUR | 100 ppm d'acétylène résiduel | 2 ppm d'acétylène résiduel |
|---|---|---|
| Palladium seul | 101,2 | 100,8 |
| Palladium + Or | 101,5 | 101,3 |

Exemple 9

Dans cet exemple, on effectue l'hydrogénation de la coupe $C_2$ de l'exemple 8 dans les conditions de l'exemple 8. Les essais sont poursuivis pendant 200 heures. Dans le tableau 8, on a indiqué la teneur résiduelle en acétylène obtenue au bout de la 5$^e$ et de la 200$^e$ heure. Là encore, on note l'effet favorable de la présence d'or.

Tableau 8

Catalyseur                                                                    Acétylène résiduel

| CATALYSEUR | 5 heures | 200 heures |
|---|---|---|
| Palladium seul | 3 | 6 |
| Palladium + Or | 3 | 3 |

## Revendications

1. Procédé d'hydrogénation sélective de l'acétylène contenu dans un mélange d'acétylène et d'éthylène dans lequel ledit mélange est mis en circulation à travers un lit fixe de catalyseur de palladium sur alumine, caractérisé en ce que l'on opère en présence d'une phase liquide à base d'au moins un hydrocarbure, ladite phase liquide renfermant 15 à 100 %, en poids de la phase liquide, d'au moins un hydrocarbure aromatique, ladite phase liquide contenant en outre au moins un composé aminé à l'état dissous dans la phase liquide et choisi dans le groupe constitué par les amines et les polyamines primaires et secondaires.

2. Procédé selon la revendication 1, dans lequel la concentration du composé aminé est de 0,01 à 10 % du poids d'hydrocarbure(s) de la phase liquide.

3. Procédé selon la revendication 1, dans lequel la concentration du composé aminé est de 0,1 à 1 % du poids de la phase liquide.

4. Procédé selon l'une des revendications 1 à 3 dans lequel le composé aminé est choisi dans le groupe constitué par la butylamine, l'éthylamine, la diéthylamine, la triéthylamine, la pipéridine, la morpholine, la pipérazine, l'éthylènediamine, la triéthylènediamine et l'éthanolamine.

5. Procédé selon l'une des revendications 1 à 4, dans lequel le composé aminé est choisi dans le groupe constitué par la pipéridine, l'éthylènediamine, l'éthanolamine, la diéthylamine et la butylamine.

6. Procédé selon l'une des revendications 1 à 5, dans lequel le catalyseur renferme 0,01 à 1 % en poids de palladium.

7. Procédé selon l'une des revendications 1 à 6, dans lequel le catalyseur renferme 0,01 à 1 % en poids de palladium et 0,01 à 1 % en poids d'argent (Ag) ou d'or (Au).

8. Procédé selon la revendication 7, dans lequel le rapport pondéral Au/Pd ou Ag/Pd est inférieur à 1.

9. Procédé selon la revendication 8, dans lequel le rapport pondéral Au/Pd est compris entre 0,05 et 0,5.

## Claims

1. A process for the selective hydrogenation of acetylene contained in a mixture of acetylene and

7

ethylene, wherein said mixture is passed through a fixed bed of palladium-on-alumina catalyst, characterized in that the hydrogenation is performed in the presence of a liquid phase comprising at least one hydrocarbon, said liquid phase containing 15 to 100 % by weight of at least one aromatic hydrocarbon and further containing at least one amine compound dissolved in the liquid phase and selected from the group consisting of primary and secondary amines and polyamines.

2. A process according to claim 1, wherein the amine compound is present at a concentration of 0.01 to 10 % by weight of hydrocarbon in the liquid phase.

3. A process according to claim 1, wherein the concentration of the amine compound is from 0.1 to 1 % by weight of hydrocarbon in the liquid phase.

4. A process according to one of claims 1 to 3, wherein the amine compound is selected from the group consisting of butylamine, ethylamine, diethylamine, triethylamine, piperidine, morpholine, piperazine, ethylenediamine, triethylenediamine and ethanolamine.

5. A process according to one of claims 1 to 4, wherein the amine compound is selected from the group consisting of piperidine, ethylenediamine, ethanolamine, diethylamine and butylamine.

6. A process according to one of claims 1 to 5, wherein the catalyst contains 0.01 to 1 % by weight of palladium.

7. A process according to one of claims 1 to 6, wherein the catalyst contains 0.01 to 1 % by weight of palladium and 0.01 to 1 % by weight of silver or gold.

8. A process according to claim 7, wherein the ratio by weight of Au/Pd or Ag/Pd is lower than 1.

9. A process according to claim 8, wherein the ratio by weight of Au/Pd is 0.05 and 0.5.


**Patentansprüche**

1. Verfahren zur selektiven Hydrierung von Acetylen, das in einer Mischung von Acetylen und Ethylen enthalten ist, wobei diese Mischung durch ein Katalysatorfestbett von Palladium auf Aluminiumoxid in Zirkulation gebracht wird, dadurch gekennzeichnet, daß man in Gegenwart einer flüssigen Phase auf der Basis von zumindest einem Kohlenwasserstoff arbeitet, wobei diese flüssige Phase 15 bis 100 Gew.-% der flüssigen Phase an zumindest einem aromatischen Kohlenwasserstoff aufweist und diese flüssige Phase außerdem zumindest eine Aminverbindung in gelöstem Zustand in der flüssigen Phase aufweist, die aus der Gruppe primäre und sekundäre Amine und Polyamine gewählt ist.

2. Verfahren nach Anspruch 1, dadurch gekennzeichnet, daß die Konzentration der Aminverbindung 0,01 bis 10 Gew.-% des Kohlenwasserstoffs oder der Kohlenwasserstoffe der flüssigen Phase beträgt.

3. Verfahren nach Anspruch 1, dadurch gekennzeichnet, daß die Konzentration der Aminverbindung 0,1 bis 1 Gew.-% der flüssigen Phase beträgt.

4. Verfahren nach einem der Ansprüche 1 bis 3, dadurch gekennzeichnet, daß die Aminverbindung aus der Gruppe Butylamin, Ethylamin, Diethylamin, Triethylamin, Piperidin, Morpholin, Piperazin, Ethylendiamin, Triethylendiamin und Ethanolamin gewählt ist.

5. Verfahren nach einem der Ansprüche 1 bis 4, dadurch gekennzeichnet, daß die Aminverbindung aus der Gruppe Piperidin, Ethylendiamin, Ethanolamin, Diethylamin und Butylamin gewählt ist.

6. Verfahren nach einem der Ansprüche 1 bis 5, dadurch gekennzeichnet, daß der Katalysator 0,01 bis 1 Gew.-% Palladium enthält.

7. Verfahren nach einem der Ansprüche 1 bis 6, dadurch gekennzeichnet, daß der Katalysator 0,01 bis 1 Gew.-% Palladium und 0,01 bis 1 Gew.-% Silber (Ag) oder Gold (Au) enthält.

8. Verfahren nach Anspruch 7, dadurch gekennzeichnet, daß das Gewichtsverhältnis Au/Pd oder Ag/Pd kleiner als 1 ist.

9. Verfahren nach Anspruch 8, dadurch gekennzeichnet, daß das Gewichtsverhältnis Au/Pd zwischen 0,05 und 0,5 liegt.